# FASCICULE DE BREVET EUROPEEN

(11) **EP 2 887 825 B1**
(45) Date de publication et mention de la délivrance du brevet: **12.08.2020**
(21) Numéro de dépôt: 13762186.8
(22) Date de dépôt: 26.08.2013
(51) Int. Cl.: A61K 35/748, A23L 3/3418, A23L 3/3463, A23L 3/3472, A23L 33/105, A23L 33/115, A23L 33/135, A23L 17/60, A23L 3/015

(54) **COMPOSITION ALIMENTAIRE À BASE DE SPIRULINE FRAÎCHE ET PROCÉDÉ DE PRÉPARATION**
NAHRUNGSMITTEL MIT FRISCHEM SPIRULINA UND HERSTELLUNGSVERFAHREN DAFÜR
FOOD COMPOSITION CONTAINING FRESH SPIRULINA AND PRODUCTION METHOD THEREOF

(30) Priorité: 27.08.2012 FR 1258003
(43) Date de publication de la demande: 01.07.2015
(73) Titulaire: Dareville, Eric, 21200 Meursanges (FR)
(72) Inventeur: Dareville, Eric, 21200 Meursanges (FR)
(74) Mandataire: Misrahi, Elie René
(86) Numéro de dépôt international: PCT/FR2013/051965
(87) Numéro de publication internationale: WO 2014/033395

(56) Documents cités:
- US-A- 5 709 855
- US-A1- 2005 281 839
- US-B1- 6 698 134
- ELIZANGELA G., O. ET AL: "Optimisation of Spirulina platensis convective drying: evaluation of phycocyanin loss and lipid oxidation", INTERNATIONAL JOURNAL OF FOOD SCIENCE AND TECHNOLOGY, vol. 45, 2010, pages 1572-1578, XP002697870,
- DATABASE WPI Week 199602 Thomson Scientific, London, GB; AN 1996-015216 XP002697871, & JP H07 289201 A (SPIRULINA KENKYUSHO KK) 7 novembre 1995 (1995-11-07)
- LIN WANG ET AL: "ANTIOXIDANT ACTIVITY OF SPIRULINA PLATENSIS EXTRACTS BY SUPERCRITICAL CARBON DIOXIDE EXTRACTION", FOOD CHEMISTRY, vol. 105, 2007, pages 36-41, XP002697872,
- J. A. MENDIOLA ET AL: "Screening of functional compounds in supercritical fluid extracts from Spirulina platensis", SCREENING OF FUNCTIONAL COMPOUNDS IN SUPERCRITICAL FLUID EXTRACT FROM SPIRULINA PLATENSIS, vol. 102, 2007, pages 1357-1367, XP002697873,
- DATABASE WPI Week 200917 Thomson Scientific, London, GB; AN 2009-F26612 XP002697874, & CN 101 356 967 A (DENG J) 4 février 2009 (2009-02-04)

## Description

### Domaine technique

La présente invention a pour objet un procédé de préparation d'une composition alimentaire à base de spiruline fraîche ainsi qu'une composition alimentaire susceptible d'être obtenue par ledit procédé.

### Technique antérieure

Par spiruline, on vise ici les espèces comestibles de cyanobactéries du genre *Arthrospira* et notamment en particulier l'espèce *Arthrospira platensis.*

La spiruline est un produit très intéressant au plan nutritionnel. En effet, elle contient 55 % à 65 % de protéines végétales. Elle contient aussi des acides gras essentiels : oméga 6, acide gamma-linolénique. La spiruline contient également des vitamines : A, E, D, B1, B2, B3, B6, B7, B8, K et bêta carotène. La spiruline est aussi constituée de minéraux et d'oligo-éléments: calcium, phosphore, magnésium, fer, zinc, cuivre, manganèse, chrome, sodium, potassium et sélénium. De plus, la spiruline est très riche en chlorophylle et en phycocyanine qui sont des pigments aux propriétés détoxifiantes, antioxydantes et anti-radicalaires remarquables. Enfin, des enzymes sont contenus dans la spiruline dont le plus important est la superoxyde dismutase (SOD) qui contient du fer.

La spiruline est donc un complément alimentaire très efficace, quelques grammes consommés quotidiennement permettant de pallier nombre de carences nutritionnelles.

De surcroit, sa culture est très intéressante au plan de la productivité et de l'écologie : une faible consommation d'eau comparativement à d'autres sources de protéines végétales ou animales et des rendements à l'hectare très élevés (50 t/ha). En outre, aucun produit phytosanitaire, pesticide ou antibiotique n'est nécessaire.

Toutefois, sa consommation et sa commercialisation sous forme fraîche n'est pas répandue, du fait qu'elle pourrit peu de temps après récolte. Ainsi, la quasi totalité de la spiruline commercialisée se présente sous forme desséchées/déshydratée, en brindilles séchées ou en poudre. Or sous cette forme, la spiruline a un goût prononcé qui peut être un frein à sa consommation. De plus, la déshydratation s'accompagne d'une baisse des qualités nutritionnelles.

La présente invention vise donc à proposer une nouvelle forme de préparation de la spiruline permettant de préserver dans la durée les caractéristiques nutritionnelles et organoleptiques de la spiruline fraîche ainsi préparée, qui soit en outre économique à mettre en œuvre et applicable à grande échelle.

### Résumé de l'invention

L'invention a pour objet un procédé de préparation d'une composition alimentaire à base de spiruline fraîche comportant au moins 50% en masse de spiruline fraîche avec une teneur en eau supérieure ou égale à 15%; 0.1 à 4% d'agents antioxydants et d'agents antimicrobiens extraits de plantes; 0.1 à 20% de corps gras alimentaire.

Ce procédé comporte les étapes suivantes :
- Récolte de la spiruline ;
- Filtration et pressage ;
- Transfert dans une enceinte de mélange et mise sous vide d'une quantité de spiruline fraîche filtrée et pressée telle qu'elle représentera au moins 50% de la masse totale finale ;
- Introduction dans l'enceinte sous vide les agents antioxydants et les agents antimicrobiens extraits de
   plantes, suivie d'un brassage ;
- Introduction dans l'enceinte sous vide le corps
   gras alimentaire, suivie d'un brassage ;
- Rupture du vide, récupération de la composition et conditionnement.

On comprend bien que le procédé de préparation de la composition évite l'oxydation de la masse de spiruline fraîche récoltée et pressée, permettant de la conserver une fois conditionnée pendant plusieurs mois à température ambiante. En outre, il s'avère avantageusement que le goût de la spiruline ainsi préparée est moins marqué que celui des préparations déshydratées et facilite ainsi sa consommation.

La présente invention concerne également une composition alimentaire à base de spiruline fraîche susceptible d'être obtenue par ledit procédé de préparation. Cette composition comporte :
- au moins 50% en masse de spiruline fraîche avec une teneur en eau supérieure ou égale à 15% ;
- 0.1 à 4 % d'agents antioxydants et d'agents antimicrobiens extraits de plantes ;
- 0.1 à 20% de corps gras alimentaire.

Par « à base de », on entend dans le présent texte que la spiruline fraîche est l'ingrédient principal et majoritaire de la composition.

D'autres avantages et caractéristiques de l'invention ressortiront mieux de la description détaillée qui va suivre.

### Description détaillée de l'invention

L'invention a donc pour premier objet un procédé de préparation d'une composition alimentaire à base de spiruline fraîche telle que décrite précédemment.

Ce procédé comporte les étapes suivantes :
- Récolte de la spiruline ;
- Filtration et pressage ; la filtration est réalisée sur filtres microporeux (30 microns) ;
- Transfert dans une enceinte de mélange et mise sous vide d'une quantité de spiruline fraîche filtrée et pressée telle qu'elle représentera au moins 50% de la masse totale finale ;
- Introduction dans l'enceinte sous vide les agents antioxydants et les agents antimicrobiens tels que
   définis précédemment, suivie d'un brassage ;
- Introduction dans l'enceinte sous vide l'huile alimentaire, suivie d'un brassage ;
- Rupture du vide, récupération de la composition et conditionnement.

La mise sous vide de l'enceinte de mélange permet de supprimer l'air au contact de la masse de spiruline et d'éviter son oxydation. Le vide peut atteindre -96kPa. Cette mise sous vide conduit à une déshydratation partielle de la spiruline, sans toutefois tomber sous une teneur en eau inférieure à 15%.

Selon une variante, ladite enceinte peut être connectée à une seconde enceinte, contenant des zéolites, de telle sorte que l'atmosphère de la première enceinte circule à l'intérieur de la deuxième enceinte. On améliore ainsi les conditions de déshydratation partielle et le retrait de l'eau superficielle. Toutefois, on notera que le temps de séjour des ingrédients dans l'enceinte est court, de quelques minutes, au maximum de quelques dizaines de minutes.

En outre, une étape facultative de rinçage à l'eau potable avant pressage peut être effectuée. Elle permet d'éliminer une partie du sel provenant du milieu de culture de la spiruline et peut être recyclée en circuit fermé grâce à une ultrafiltration, une décontamination par irradiation UV et une nouvelle calibration. Préférentiellement, ledit rinçage est effectué avec de l'eau stérile. Encore plus préférentiellement, les paramètres de pH, de salinité et d'alcalinité de ladite eau stérile sont contrôlées.

Selon une variante préférée, le conditionnement est réalisé sous vide, par exemple par jet de vapeur pour des pots de verre capsulés ou avec une machine d'emballage sous vide pour sachets soudables, optionnellement sous atmosphère protectrice de gaz neutre, du type CO2 ou azote.

Selon une autre variante avantageuse, le conditionnement met en œuvre une pascalisation, c'est-à-dire un traitement à très haute pression. Le recours à ce traitement permet avantageusement de réduire drastiquement la teneur en corps gras et en agents antimicrobiens et antioxydants présents dans la composition, à moins d'1% en poids chacun, jusqu'à 0.1%.

On obtient ainsi une préparation alimentaire à base de spiruline fraîche qui se conserve au moins 12 mois à température ambiante, sans altération des qualités organoleptiques de la composition.

L'invention a également pour objet une composition alimentaire à base de spiruline fraîche susceptible d'être obtenue par ledit procédé. Cette composition comporte :
- au moins 50% en masse de spiruline fraîche avec une teneur en eau supérieure ou égale à 15% ;
- 0.1 à 4 % d'agents antioxydants et d'agents antimicrobiens extraits de plantes ;
- 0.1 à 20% de corps gras alimentaire.

Par spiruline fraîche, on entend une spiruline récoltée moins de 12h auparavant, non congelée, non séchée ; selon le procédé de préparation, cette spiruline fraîche est filtrée et pressée et comporte une teneur en eau strictement supérieure ou égale à 15%.

La composition peut aussi comporter un autre ingrédient, végétal ou animal, selon le goût et la texture recherchés : le total d'ingrédients principaux atteint 75 à 99 % en poids, complétés d'huile ou corps gras et d'extraits de plantes aux propriétés antioxydant et/ou antimicrobien.

De préférence, la composition comporte au moins 80% de spiruline fraîche. De manière très préférée, elle contient au moins 85% de spiruline.

Selon une première caractéristique, les extraits de plante remplissant la fonction d'agent antioxydant sont pris parmi : extrait de romarin, extrait de sauge, extrait de fenugrec, extrait de basilic sacré, extrait de trèfle rouge, extrait de galanga, extrait de gingembre, extrait de ginseng, licopène de tomate. De préférence, l'extrait est un extrait de romarin.

Selon une autre caractéristique, les extraits de plante remplissant la fonction d'agent antibactérien sont pris parmi : extraits et/ou huiles essentielles d'origan (Origanum vulgare, Origanum compactum), des thyms vulgaires (Thymus vulgaris), de marjolaine (Origanum majorana), de cannelle feuille ou écorce (Cinnamomum zeylanicum ou Cinnamomum verum), de clou de girofle (Syzygium aromaticum), de coriandre (Coriandrum sativum), d'ail, d'extraits oléorésineux des Capsicum, de barbe de Jupiter (Usnea barbata), extrait de poivre, huile essentielle de Tea tree (Melaleuca alternifolia), épices, moutarde, wasabi. Notamment, les agents antibactériens sont des dérivés soufrés de l'alliicine extraits de plantes du genre Allium, et plus précisément des variétés Allium sativum et Allium cepa. De préférence, les extraits sont des extraits d'origan, de thym, de marjolaine, de cannelle feuille, de clou de girofle et de coriandre.

Selon une dernière caractéristique, le corps gras alimentaire est de préférence une huile alimentaire. On pourra également mettre en œuvre une seule huile ou un mélange de différentes huiles et le cas échéant de graisses animales (beurre, saindoux) sans sortir du cadre de la présente invention. De préférence, on choisira une huile alimentaire riche en oméga 3 : ceci permet avantageusement de conférer à la composition alimentaire un ratio oméga 3/oméga 6 plus équilibré, du fait de la teneur en oméga 6 de la spiruline. Ainsi, l'huile alimentaire est de préférence prise seule ou en combinaison parmi huile de chia, huile de lin, huile de cameline, huile de colza, huile de poisson ou toutes autres sources d'huile ou corps gras alimentaires riches en oméga 3. De préférence, l'huile est de l'huile de chia.

De préférence, la proportion en corps gras, et notamment en huile, est inférieure ou égale à 5% en poids.

### Application industrielle

La composition et son procédé d'obtention peuvent être mis en œuvre dans des installations agroalimentaires à proximité immédiate des zones de production et de récolte de spiruline.

## Revendications

1. Procédé de préparation d'une composition alimentaire à base de spiruline fraîche, comportant au moins 50% en masse de spiruline fraîche avec une teneur en eau supérieure ou égale à 15%; 0.1 à 4% d'agents antioxydants et d'agents antimicrobiens extraits de plantes; 0.1 à 20% de corps gras alimentaire; **caractérisé en ce qu'**il comporte les étapes
suivantes :
• Récolte de la spiruline ;
• Filtration et pressage ;
• Transfert dans une enceinte de mélange et mise sous vide d'une quantité de spiruline filtrée et pressée telle qu'elle représentera au moins 50% de la masse totale finale ;
• Introduction dans l'enceinte sous vide les agents antioxydants et les agents antimicrobiens , suivie d'un brassage ;
• Introduction dans l'enceinte sous vide le corps gras alimentaire, suivie d'un brassage;
• Rupture du vide, récupération de la composition et conditionnement.

2. Procédé de préparation d'une composition alimentaire à base de spiruline fraîche selon la revendication précédente **caractérisé en ce qu'**il comporte une étape de rinçage à l'eau potable avant le pressage.

3. Procédé de préparation d'une composition alimentaire à base de spiruline fraîche selon l'une des revendications 1 à 2 **caractérisé en ce que** le conditionnement est sous vide.

4. Procédé de préparation d'une composition alimentaire à base de spiruline fraîche selon l'une des revendications 1 à 3 **caractérisé en ce que** le conditionnement met en œuvre une pascalisation.

5. Composition alimentaire à base de spiruline fraîche **susceptible d'être obtenue par le procédé** selon l'une quelconque des revendications précédentes **caractérisée en ce qu'**elle comporte :
- au moins 50% en masse de spiruline fraîche avec une teneur en eau supérieure ou égale à 15% ;
- 0.1 à 4 % d'agents antioxydants et d'agents antimicrobiens extraits de plantes ;
- 0.1 à 20% de corps gras alimentaire.

6. Composition selon la revendication 5 **caractérisée en ce qu'**elle comporte au moins 80% de spiruline fraîche.

7. Composition selon l'une des revendications 5 et 6 **caractérisée en ce que** les extraits de plante remplissant la fonction d'agent antioxydant sont pris parmi : extrait de romarin, extrait de sauge, extrait de fenugrec, extrait de basilic sacré, extrait de trèfle rouge, extrait de galanga, extrait de gingembre, extrait de ginseng, licopène de tomate.

8. Composition selon l'une des revendications 5 à 7 **caractérisé en ce que** les extraits de plante remplissant la fonction d'agents antibactérien sont pris parmi : extraits et/ou huiles essentielles d'origan (Origanum vulgare, Origanum compactum), des thyms vulgaires (Thymus vulgaris), de marjolaine (Origanum majorana), de cannelle feuille ou écorce (Cinnamomum zeylanicum ou Cinnamomum verum), de clou de girofle (Syzygium aromaticum), de coriandre (Coriandrum sativum), d'ail, d'extraits oléorésineux des Capsicum, de barbe de Jupiter (Usnea barbata), extrait de poivre, huile essentielle de Tea tree (Melaleuca alternifolia), épices, moutarde, wasabi.

9. Composition selon l'une des revendications 5 à 8 **caractérisée en ce que** le corps gras alimentaire est une huile alimentaire prise seule ou en combinaison parmi : huile de chia, huile de lin, huile de cameline, huile de colza, huile de poisson ou toutes autres sources d'huile ou corps gras alimentaires.

## Patentansprüche

1. Verfahren zur Herstellung einer
Nahrungsmittelzusammensetzung auf Basis von frischer Spirulina, umfassend mindestens 50 Massen-% frischer Spirulina mit einem Wassergehalt von größer oder gleich 15 %; 0,1 bis 4 % Antioxidantien und antimikrobielle Mittel, extrahiert aus Pflanzen; 0,1 bis 20 % Fettnahrungsmittel, **gekennzeichnet durch** folgende Schritte:
- Ernte von Spirulina;
- Filtration und Pressen;
- eine Menge gefilterter und gepresster Spirulina in eine Mischkammer geben und unter Vakuum stellen, die mindestens 50 % der endgültigen Gesamtmasse ausmacht;
- Einbringen von antioxidativen und antimikrobiellen Mitteln in die Vakuumkammer, gefolgt von Mischen
- Einfüllen von Fettnahrungsmittel in die Vakuumkammer und anschließendes Mischen;
- Unterbrechen des Vakuums, Rückgewinnung der Zusammensetzung und Verpackung.

2. Verfahren zur Herstellung einer
Nahrungsmittelzusammensetzung auf Basis von frischer Spirulina nach dem vorhergehenden Anspruch, **dadurch gekennzeichnet, daß** es einen Spülschritt mit Trinkwasser vor dem Pressen umfasst.

3. Verfahren zur Herstellung einer
Nahrungsmittelzusammensetzung auf der Basis von frischer Spirulina nach einem der Ansprüche 1 bis 2, **dadurch gekennzeichnet, daß** die Verpackung unter Vakuum erfolgt.

4. Verfahren zur Herstellung einer
Nahrungsmittelzusammensetzung auf Basis von frischer Spirulina nach einem der Ansprüche 1 bis 3, **dadurch gekennzeichnet, daß** die Verpackung eine Pascalisation verwendet.

5. Nahrungsmittelzusammensetzung auf Basis von frischer Spirulina, erhältlich durch das Verfahren nach einem der vorhergehenden Ansprüche, **dadurch gekennzeichnet, daß** sie folgende Bestandteile enthält:
- mindestens 50 Gewichtsprozent an frische Spirulina mit einem Wassergehalt von 15 % oder mehr;
- 0,1 bis 4 % der aus Pflanzen extrahierten antioxidativen und antimikrobiellen Mittel;
- 0,1 bis 20 % Fettnahrungsmittel.

6. Zusammensetzung nach Anspruch 5, **dadurch gekennzeichnet, daß** sie mindestens 80 % frische Spirulina enthält.

7. Zusammensetzung nach einem der Ansprüche 5 und 6,
**dadurch gekennzeichnet, daß** Pflanzenextrakte, die als Antioxidantien wirken, ausgewählt sind aus : Rosmarinextrakt, Salbeiextrakt, Extract aus Bockshornklee, Extrakt aus heiligem Basilikum, Extrakt aus Rotklee, Extrakt aus Galanga, Extrakt aus Ingwer, Extrakt aus Ginseng, Tomaten Licopene.

8. Zusammensetzung nach einem der Ansprüche 5 bis 7, **dadurch gekennzeichnet, daß** Pflanzenextrakte, die als antibakterielle Mittel wirken, ausgewählt sind aus : Extrakten und/oder ätherischen Ölen von Oregano (Origanum vulgare, Origanum compactum), Vulgar-Thymian (Thymus vulgaris), Marjoram (Origanum majorana), Zimtblätter oder Rinde (Cinnamomum zeylanicum oder Cinnamomum verum), Gewürtsnelke (Syzygium aromaticum), Koriander (Coriandrum sativum), Knoblauch, Oleoresin Extrakten aus Capsicum, Jupiter's beard (Usnea barbata), Pfeffer-Extract, ätherisches Öl (Melaleuca alternifolia), Gewürze, Senf, Wasabi.

9. Zusammensetzung nach einem der Ansprüche 5 bis 8, **dadurch gekennzeichnet, daß** das Fettnahrungsmittel ein Nahrungsmittelöl ist, allein oder in Kombination, ausgewählt aus: Chiaöl, Leinöl, Kamelinöl, Rapsöl, Fischöl oder einer anderen Öl- oder Fettnahrungsquelle.

## Claims

1. Process of preparation of a food composition based on fresh spirulina, comprising at least 50% by mass of fresh spirulina with a water content greater than or equal to 15%; 0.1 to 4% of antioxidant and antimicrobial agents extracted from plants; 0.1 to 20% of food fat **characterised in that** it comprises the following steps :
- harvesting of spirulina;
- filtration and pressing;
- transfer into a mixing chamber and place under vacuum a quantity of filtered and pressed spirulina that will represent at least 50% of the final total mass;
- introduction in the vacuum chamber of antioxidant agents and antimicrobial agents, followed by mixing
- introduction of food fat in the vacuum chamber, followed by mixing;
- vacuum breaking, composition recovery and packaging.

2. Process for the preparation of a food composition based on fresh spirulina according to the previous claim, **characterised in that** it comprises a rinsing step with potable water before pressing.

3. Process for preparing a food composition based on fresh spirulina according to anyone of claims 1 to 2, **characterised in that** the packaging is carried out under vacuum.

4. Process for preparing a food composition based on fresh spirulina according to anyone of claims 1 to 3, **characterised in that** the packaging makes use of a pascalisation.

5. Food composition based on fresh spirulina **obtainable by the process** according to any of the preceding claims,
**characterised in that** it comprises :
- at least 50% by mass of fresh spirulina with a water content of 15% or more;
- 0.1 to 4% of antioxidant and antimicrobial agents extracted from plants;
- 0.1 to 20% of food fat.

6. Composition according to Claim 5, **characterised in that** it comprises at least 80% of fresh spirulina.

7. Composition according to anyone of claims 5 and 6, **characterised in that** plant extracts acting as antioxidants are chosen among : rosemary extract, sage extract, fenugreek extract, sacred basil extract, red clover extract, galanga extract, ginger extract, ginseng extract, tomato licopene.

8. Composition according to anyone of claims 5 to 7, **characterised in that** plant extracts acting as antibacterial agents are chosen among : extracts and/or essential oils of oregano (Origanum vulgare, Origanum compactum), vulgar thyme (Thymus vulgaris), marjoram (Origanum majorana), cinnamon leaf or bark (Cinnamomum zeylanicum or Cinnamomum verum), clove (Syzygium aromaticum), coriander (Coriandrum sativum) , garlic, Capsicum oleoresinous extracts, Jupiter's beard (Usnea barbata), pepper extract, Tea tree essential oil (Melaleuca alternifolia), spices, mustard, wasabi.

9. Composition according to anyone of claims 5 to 8, **characterised in that** the fat food is a food oil, taken alone or in combination, chosen among : chia oil, linseed oil, cameline oil, rapeseed oil, fish oil or any other sources of oil or fat food.
